# EUROPEAN PATENT SPECIFICATION

(11) **EP 3 412 177 B1**
(45) Date of publication and mention of the grant of the patent: **27.09.2023**
(21) Application number: 17746894.9
(22) Date of filing: 24.01.2017
(51) Int. Cl.: A61G 7/05, A61B 5/00, A47C 21/00

(54) **INTELLIGENT ELECTRIC BED SYSTEM**
INTELLIGENTES ELEKTRISCHES BETTSYSTEM
SYSTÈME DE LIT ÉLECTRIQUE INTELLIGENT

(30) Priority: 02.02.2016 CN 201610071895
(43) Date of publication of application: 12.12.2018
(73) Proprietor: Keeson Technology Corporation Limited, Jiaxing, Zhejiang 314016 (CN)
(72) Inventor: SHAN, Huafeng, Jiaxing, Zhejiang 314016 (CN); CAO, Hui, Jiaxing, Zhejiang 314016 (CN); YU, Qun, Jiaxing, Zhejiang 314016 (CN)
(74) Representative: Viering, Jentschura & Partner mbB Patent- und Rechtsanwälte
(86) International application number: PCT/CN2017/072418
(87) International publication number: WO 2017/133574

(56) References cited:
- EP-A1- 3 377 019
- CN-A- 104 138 297
- CN-A- 104 188 638
- CN-A- 104 188 638
- CN-U- 204 306 471
- CN-U- 204 411 155
- CN-U- 204 411 155
- CN-U- 204 636 331
- JP-A- 2005 000 411
- TW-A- 201 417 793
- US-A1- 2006 235 283
- US-A1- 2013 245 389
- US-A1- 2014 323 816
- US-A1- 2015 059 096
- US-A1- 2015 164 721

## Description

### Field of Technology

The present application relates to an intelligent electric bed system.

### Background Technology

The patent application TW 201 417 793 A discloses a medical monitoring system which is applied to a hospital bed. The medical monitoring system enables a medical staff (manager) to automatically track and monitor the hospital bed through a setting of a system interface, and thereby effectively grasping the patient. For this purpose the medical monitoring system comprises: a motor control unit for detecting information of a working position of a motor device disposed on the hospital bed, and driving the motor device to change the bed posture of the hospital bed a weight measuring device disposed on the bed for sensing the weight information of the user; a bed monitoring device coupled or adjacent to the bed and coupled to the motor control unit and the weighing device, respectively; a sensing unit configured to process information received from the motor control unit and the weight measuring device, and to transmit a control signal to the motor control unit to drive the motor device to change the bed posture; and a management station which monitors the computer, and the signal connection. The management station's monitoring computer includes a manager interface for presenting the current state of the bed and for allowing the administrator to operate the hospital bed and to communicate with the patient.

Further intelligent bed systems are known from:
the patent application EP 3 377 019 A1 disclosing an adjustable bedframe and operating methods thereof;
the patent application US 2014/323816 A1 referring to a system for patient support, monitoring and treatment;
the patent application US 2006/235283 A1 disclosing a comfort suit for an intelligent patient bed;
the patent application CN 104 188 639 A depicting a sleep guaranteeing system which also comprises a bedding;
the patent application CN 204 411 155 U referring to a rehabilitation bed monitoring system and a rehabilitation bed;
the patent application US 2015/059096 A1 disclosing a communication system adapted to communicate between a handheld remote control and a first adjustable bed controller of a first adjustable bed facility, and a second communication system adapted to communicate between the first adjustable bed controller of the first adjustable bed facility and a second adjustable bed controller of a second adjustable bed facility;
the patent application US 2013/245389 A1 disclosing a patient monitoring and intervention system, comprising an interface for receiving data representing multiple different parameters from multiple different sensors, comprising sensors in a patient bed and attached to a patient; and
   the patent application US 2015/164721 A1 disclosing a sleeping-posture-control bed system including a bed floor including a back-lifting portion for lifting the user's back.

An electric bed includes a plurality of bed planks hinged to each other, a drive motor configured to drive the plurality of bed planks to raise or rotate, and a linkage mechanism.

An existing electric bed uses a terminal device to adjust posture of the electric bed. For this, an additional terminal device mating therewith is required for each electric bed. In mass production, remote controllers may cause additional production cost. At the same time, it is possible that a remote controller mating with the electric bed is lost and/or damaged. If such a situation occurs, a user will be temporarily unable to adjust the electric bed, thereby causing a lot of inconvenience.

In addition, the existing terminal device can only send a preset execution command to the electric bed according to the remote control of the user, but cannot make changes for different usages; the terminal device having this single function cannot meet the requirements of users. For example, when sleeping condition of a user changes, the electric bed cannot intervene the user's sleep in time, and thus fails to effectively improve the sleeping condition of the user.

### Summary

It is to be noted that the purpose of the present application is to overcome one or more of the disadvantages that have been found in the prior art.

For this purpose, an intelligent electric bed system according to claim 1 is provided, comprising an electric bed, a terminal device and a server. The dependent claims show further embodiments of a suchlike intelligent bed system.

The electric bed, adapted to monitor physical conditions of a user in real time and adjust a state of the electric bed based on monitoring data, includes:
a monitoring module, configured to monitor the physical conditions of the user, and send the monitoring data to a wireless communication module; and
a main control module, configured to receive a control signal from the wireless communication module, and adjust the state of the electric bed based on the control signal;
wherein the wireless communication module transmits the received monitoring data to the server, receives the control signal from the server, and sends the control signal to the main control module; and the server generates the control signal based on a setting signal sent by the terminal device and the monitoring data.

Further, the main control module sends information data including the state of the electric bed to the wireless communication module, and the wireless communication module sends the information data to the server.

Further, a connection mean of the wireless communication module includes at least one selected from the group consisting of Wi-Fi, Bluetooth, infrared, and ZigBee.

Further, the monitoring data includes at least one selected from the group consisting of a heart rate, a respiratory rate, an intensity of snore, an intensity of respiration, and numbers of turning and tossing in bed.

Further, the state of the electric bed includes at least one selected from the group consisting of an inclination angle of each bed plank, a state of a drive motor, and a state of an indicator light.

Further, the bed plank includes at least one selected from the group consisting of a head bed plank, a leg bed plank, a haunch bed plank, and a waist bed plank.

Further, the server is a cloud server.

The terminal device, adapted to adjust a state of an electric bed via a server, wherein the terminal device sends a setting signal to the server; the server generates, based on the setting signal, a control signal for adjusting the state of the electric bed, and further sends the control signal to the electric bed.

Further, the terminal device receives from the server and displays monitoring data indicating physical conditions of a user of the electric bed.

Further, the terminal device receives from the server and displays information data including the state of the electric bed.

Further, the terminal device communicates with the server by means of at least one connection mean selected from the group consisting of Wi-Fi, 2G, 3G, and 4G

Further, the monitoring data includes at least one selected from the group consisting of a heart rate, a respiratory rate, an intensity of snore, an intensity of respiration, and numbers of turning and tossing in bed.

Further, the state of the electric bed includes at least one selected from the group consisting of an inclination angle of each bed plank, a state of a drive motor, and a state of an indicator light.

By performing related setting with respect to the electric bed on the terminal device provided in the present application, the user may conveniently control the electric bed. Meanwhile, the user may get to know the current state of the electric bed intuitively and in real time since the terminal displays the information data of the state of the electric bed, such that the user may make more proper adjustment.

In the intelligent electric bed system provided in the present application, various conditions for changing the state of the electric bed may be preset in the server using the terminal device. The electric bed may keep connected to the server in real time, and may send monitoring data in respect of physical conditions of a user to the server. When the monitoring data matches the conditions for changing the state of the electric bed, the server may send a control signal to the electric bed to change the state of the electric bed. By using the electric bed system, changes can be made in time for usages of different users, thereby improving sleeping condition of the users.

### Brief Description of the Drawings

It should be understood that in the present application, all features, modifications, and/or specific embodiments may be combined in various combinations, except in the cases of obvious contradictions and incompatibilities.

By reading the following non-limiting illustrative embodiments, and in conjunction with the drawings, other features and advantages of the present application will become apparent. In the figures:
- Figure 1 is a schematic structural view showing an intelligent electric bed system according to the present application; and
- Figure 2 is a flow chart showing the control of an intelligent electric bed system according to the present application.

### Detailed Description

The present application will be described in detail below with reference to the embodiments and the accompanying drawings.

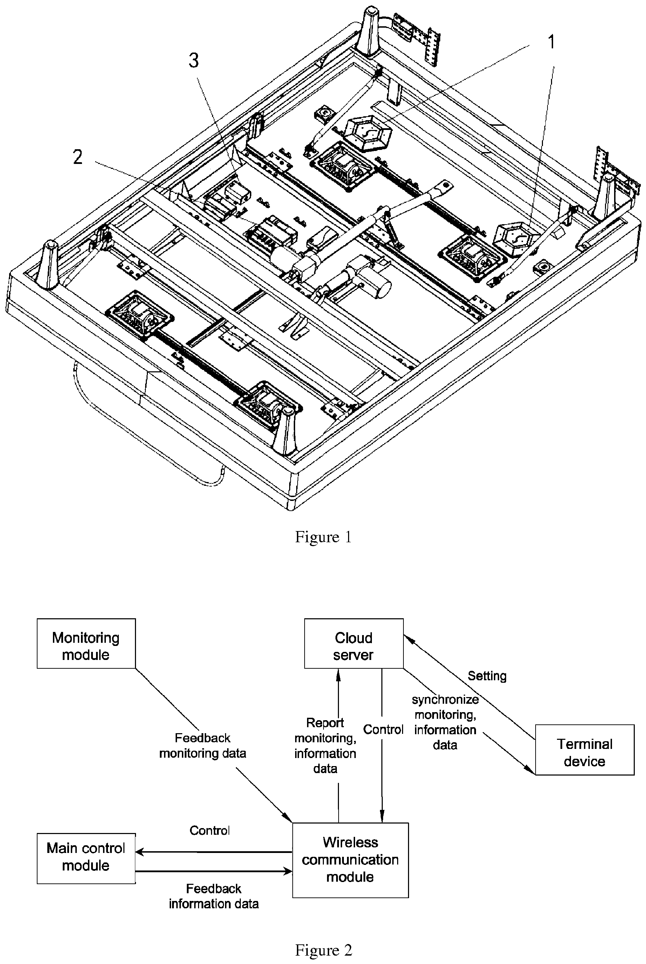

With reference to Figure 1, an intelligent electric bed system according to an embodiment of the present application is shown.

An electric bed may include a plurality of bed planks hinged to each other, a linkage mechanism connected to the plurality of bed planks, and a drive motor for driving the linkage mechanism. The electric bed may further include a monitoring module 1, a main control module 2, and a wireless communication module 3.

The monitoring module 1 may be configured to monitor physical conditions of a user, and may include a body temperature monitoring module, a heart rate monitoring module, a respiratory rate detector, a decibel meter, and the like. The monitoring module 1 may at least acquire monitoring data of physical conditions of a user. The monitoring data may include a heart rate, a respiratory rate, an intensity of snore, an intensity of respiration, numbers of turning and tossing in bed, and the like of the user. The monitoring module 1 may be adapted to send the monitoring data to the wireless communication module 3. The wireless communication module 3 may send the received monitoring data to a server. The server may store the monitoring data. Optionally, the server may send the monitoring data to a terminal device, such that the terminal device may display the monitoring data indicating the physical conditions of the user of the electric bed.

The monitoring module 1 may be mounted on an upper part of a bed body of the electric bed that is close to a heart of the user so that accurate data may be acquired.

The monitoring module 1 can monitor the heart rate, the respiratory rate, the intensity of snore, the intensity of respiration, and numbers of turning and tossing in bed of the user.

The heart rate, the respiratory rate, and the intensity of respiration may be used to determine a basic physical condition of the user.

Numbers of turning and tossing in bed may be used to determine a basic sleeping condition of the user.

By monitoring the intensity of snore, it is possible to intervene sleeping of the user in real time so that symptoms of snoring may be effectively relieved.

To avoid electromagnetic interference to the monitoring module 1 generated by wires, in one embodiment, the monitoring module 1 may communicate with the wireless communication module 3 by means of RF (Radio Frequency) wireless communication.

The main control module 2 may be electrically connected to the monitoring module 1, the wireless communication module 3, and the drive motor. A control host may be adapted to receive and analyze a control signal received by the wireless communication module 3, and control the drive motor or other devices in the electric bed according to the control signal in order to adjust a state of the electric bed. The state of the electric bed may include an inclination angle of each bed plank, a state of the drive motor, a state of an indicator light, and the like. Wherein, the bed plank may include at least one selected from the group consisting of a head bed plank, a leg bed plank, a haunch bed plank, and a waist bed plank.

In one embodiment, the main control module 2 may adjust control (including a rotation speed and number of revolutions of a spindle of the drive motor, and the like) of the drive motor, based on the control signal from the wireless communication module 3.

The main control module 2 may make various adjustments to the state of the electric bed in real time based on the control signal so to improve the sleeping condition of the user effectively. For example, when the monitoring module 1 detects that the user is snoring, the main control module 2 may raise the waist bed plank of the electric bed to a certain angle according to a preset setting. In this way, it is possible to avoid worsening the block of an airway due to relaxation of a tongue, a soft palate and a uvula, and effectively relieve the symptoms of snoring of the user. Therefore, the sleeping condition of the user may be effectively improved.

In one embodiment, the main control module 2 may further send information data including the state of the electric bed to the wireless communication module 3.

The wireless communication module 3 may be a Wi-Fi module, and, depending on cases, may also be a Bluetooth module, an infrared module, a ZigBee module, and the like, in order to wirelessly connect with the server. The wireless communication module 3 may be adapted to send the received monitoring data to the server, receive the control signal sent by the server and forward the control signal to the main control module 2, such that the control host may determine the operation to be performed.

In one embodiment, the wireless communication module 3 may receive the information data sent by the main control module 2, and further forward the information data to the server. The state of the electric bed may include an inclination angle of each bed plank, the state of the motor, the state of an indicator light, and the like.

The server may respectively and wirelessly connected to the wireless communication module 3 of the electric bed and the terminal device by means of at least one connection mean selected from the group consisting of Wi-Fi, Bluetooth, infrared, and ZigBee. The server may generate the control signal based on a setting signal sent by the terminal device and the monitoring data.

More specifically, a processing program related to the state of the electric bed may be installed in the server. On one hand, the server may receive the monitoring data of the electric bed; on the other hand, the server may receive the setting signal sent by the terminal device. The processing program may determine whether to send the control signal to the wireless communication module 3 of the electric bed, based on the monitoring data and the setting signal. For example, a threshold range of the heart rate in the terminal device may be preset to approximately 60 times/min to approximately 140 times/min, and terminal device may send the related setting signal to the server. When the monitoring data received by the server are beyond the range, the server may send the control signal to the wireless communication module 3 to adjust the state of the electric bed, for example, by raising the head bed plank or turning on a bed lamp. On the contrary, if the monitoring data falls within the threshold range, the server will not send any control signal. For another example, a floor lamp may be preset through the terminal device to be automatically turned on when the user is out of bed after 22:00, and the terminal device may send the related setting signal to the server. When receiving the user's off-bed data, the server may send the control signal to the wireless communication module 3 to turn on the floor lamp.

Optionally, the server may further store all feature parameters (such as the heart rate, the respiratory rate, the intensity of snore, the intensity of respiration, numbers of turning and tossing in bed) of the user, in order to analyze the sleeping condition of the user, thereby making a more accurate plan to improve the sleeping quality of the user.

Optionally, the server may receive the monitoring data indicating the physical conditions of the user of the electric bed from the wireless communication module 3, and further forward the monitoring data to the terminal device.

Optionally, the server may receive the information data including the state of the electric bed from the wireless communication module 3, and further forward the information data to the terminal device.

Optionally, the server may be a cloud (computing) server.

The terminal device may include a mobile phone, a tablet computer, a notebook computer, and the like. The terminal device may be installed with an application (App) and/or software configured to control the electric bed. Using the installed application (App) and/or software, the terminal device may control a wireless module inside the terminal device to transmit the setting signal to the server. Optionally, the terminal device may communicate with the server by means of at least one connection mean selected from the group consisting of Wi-Fi, 2G, 3G, and 4G

This type of application (App) and/or software may include a human-computer interaction interface including an option setting interface for user's selection. The user may customize various setting information of the electric bed via the interface, and further send the setting signal to the server. The setting signal may include thresholds of various monitoring data: the heart rate, the respiratory rate, the intensity of snore, the intensity of respiration, and numbers of turning and tossing in bed of the user. The user may select the information to be set by clicking, double clicking, sliding, touching, and inputting values.

Optionally, the interaction interface may include a display interface that provides the user information data including the state of the electric bed. The state of the electric bed may include at least one selecting from the group consisting of the inclination angle of each bed plank, the state of the motor, and the state of an indicator light.

Optionally, the interaction interface may include a display interface that provides the user the monitoring data indicating the physical conditions of the user. The monitoring data may include at least one selecting from the group consisting of the heart rate, the respiratory rate, the intensity of snore, the intensity of respiration, and numbers of turning and tossing in bed.

The terminal device may receive the above information data and the monitoring data from the server.

As shown in Figure 2, a flow chart showing the control of an intelligent electric bed system is shown.

On one hand, the monitoring module 1 of the electric bed may monitor various physical conditions of the user on the bed, and send the monitoring data indicating the physical conditions of the user to the wireless communication module 3 of the electric bed. The wireless communication module 3 may send the monitoring data to the server.

On the other hand, the terminal device may transmit the setting signal to the server. The setting signal may include the thresholds of various monitoring data.

The server may determine, via the processing program, whether to generate the control signal based on the monitoring data and the setting signal, and send the control signal to the wireless communication module 3. More specifically, when the monitoring data are beyond the set thresholds, the control signal will be sent; otherwise, no control signal will be sent. After that, the wireless communication module 3 may send the control signal to the main control module 2 so to adjust the state of the electric bed.

Optionally, the server may send the monitoring data to the terminal device, such that the terminal device may display the monitoring data.

Optionally, the main control module 2 may send the information data including the state of the electric bed to the wireless communication module 3, and the wireless communication module 3 may further send the information data to the server. The server may send the information data to the terminal device, such that the terminal device may display the information data.

The above embodiments are merely examples and do not limit the scope of the present application. Based on this, those skilled in the art can envision other embodiments that can achieve the same function within the scope of the claims of the present application.

Various embodiments and various modifications and improvements will be apparent to those skilled in the art. In particular, it should be understood that the above-described features, modifications, and/or embodiments of the present application may be combined with each other, except in the case of obvious contradictions or incompatibilities. All of these embodiments, as well as variations and modifications, are within the scope of the present application.

## Claims

1. An intelligent electric bed system, comprising an electric bed, a terminal device, a server and a wireless communication module;
wherein the electric bed is adapted to monitor physical conditions of a user in real time and adjust a state of the electric bed based on monitoring data, the electric bed comprising:
a monitoring module (1), configured to monitor the physical conditions of the user, and send the monitoring data to the wireless communication module (3); and
a main control module (2), configured to receive a control signal from the wireless communication module (3), and adjust the state of the electric bed based on the control signal;
wherein the wireless communication module (3) transmits the received monitoring data to the server, receives the control signal from the server, and sends the control signal to the main control module (2); and
**characterized in that**
the server generates the control signal based on a setting signal sent by the terminal device and the monitoring data; and
wherein the terminal device is adapted to adjust the state of an electric bed via the server, wherein the terminal device sends the setting signal to the server;
wherein the server generates, based on the setting signal, the control signal for adjusting the state of the electric bed, and further sends the control signal to the electric bed.

2. The electric bed system of claim 1, wherein the main control module (2) sends information data comprising the state of the electric bed to the wireless communication module (3), and the wireless communication module (3) sends the information data to the server.

3. The electric bed system of claim 1, wherein a connection mean of the wireless communication module (3) comprises at least one selected from the group consisting of Wi-Fi, Bluetooth, infrared, and ZigBee.

4. The electric bed system of claim 1, wherein the monitoring data comprises at least one selected from the group consisting of a heart rate, a respiratory rate, an intensity of snore, an intensity of respiration, and numbers of turning and tossing in bed.

5. The electric bed system of claim 1, wherein the state of the electric bed comprises at least one selected from the group consisting of an inclination angle of each bed plank, a state of a drive motor, and a state of an indicator light.

6. The electric bed system of claim 5, wherein the bed plank comprises at least one selected from the group consisting of a head bed plank, a leg bed plank, a haunch bed plank, and a waist bed plank.

7. The electric bed system of claim 1, wherein the server is a cloud server

8. The electric bed system of any of claims 1-7, wherein the terminal device receives from the server and displays monitoring data indicating physical conditions of a user of the electric bed.

9. The electric bed system of any of claims 1-7, wherein the terminal device receives from the server and displays information data comprising the state of the electric bed.

10. The electric bed system of any of claims 1-7, wherein the terminal device communicates with the server by means of at least one connection mean selected from the group consisting of Wi-Fi, 2G, 3G, and 4G

11. The electric bed system of claim 8, wherein the monitoring data comprises at least one selected from the group consisting of a heart rate, a respiratory rate, an intensity of snore, an intensity of respiration, and numbers of turning and tossing in bed.

12. The electric bed system of claim 9, wherein the state of the electric bed comprises at least one selected from the group consisting of an inclination angle of each bed plank, a state of a drive motor, and a state of an indicator light.

## Patentansprüche

1. Ein intelligentes elektrisches Bettsystem, das ein elektrisches Bett, ein Endgerät Vorrichtung, einen Server und ein drahtloses Kommunikationsmodul;
wobei das elektrische Bett angepasst ist, um physische Bedingungen eines Benutzers in Echtzeit zu überwachen und einen Zustand des elektrischen Bettes basierend auf Überwachungsdaten einzustellen, wobei das elektrische Bett umfasst: ein Überwachungsmodul (1), das so konfiguriert ist, dass es die physischen Bedingungen des Benutzers überwacht und die Überwachungsdaten zu dem drahtlosen Kommunikationsmodul (3) sendet; und ein Hauptsteuermodul (2), das so konfiguriert ist, dass es ein Steuersignal von dem drahtlosen Kommunikationsmodul (3) empfängt und den Zustand des elektrischen Bettes basierend auf dem Steuersignal einstellt;
wobei das drahtlose Kommunikationsmodul (3) die empfangenen Überwachungsdaten an den Server sendet, das Steuersignal vom Server empfängt und das Steuersignal an das Hauptsteuermodul (2) sendet; und
dadurch charakterisiert, dass
der Server das Steuersignal auf der Grundlage eines von der Vorrichtung gesendeten Einstellsignals und der Überwachungsdaten erstellt; und
wobei die Vorrichtung dazu eingerichtet ist, den Zustand eines elektrischen Bettes über den Server einzustellen, wobei die Vorrichtung das Einstellsignal an den Server sendet;
wobei der Server aufgrund des Einstellsignals das Steuersignal zum Einstellen des Zustands des elektrischen Bettes erstellt und das Steuersignal weiter an das elektrische Bett sendet.

2. Elektrisches Bett -system
nach Anspruch 1, wobei das Hauptsteuermodul (2) Informationsdaten, die den Zustand des elektrischen Bettes umfassen, an das drahtlose Kommunikationsmodul (3) sendet, und das drahtlose Kommunikationsmodul (3) die Informationsdaten an den Server sendet.

3. Elektrisches Bett -system
nach Anspruch 1, wobei ein Verbindungsmittel des drahtlosen Kommunikationsmoduls (3) mindestens eines aus der Gruppe bestehend aus Wi-Fi, Bluetooth, Infrarot und ZigBee umfasst.

4. Elektrisches Bett -system
nach Anspruch 1, wobei die Überwachungsdaten mindestens einen Wert umfassen, der aus der Gruppe ausgewählt ist, die aus einer Herzfrequenz, einer Atemfrequenz, einer Intensität des Schnarchens, einer Atmungsintensität und einer Anzahl von Drehungen und Wenden im Bett besteht.

5. Elektrisches Bett -system
nach Anspruch 1, wobei der Zustand des elektrischen Bettes mindestens eines umfasst, das aus der Gruppe ausgewählt ist, die aus einem Neigungswinkel jeder Bettplanke, einem Zustand eines Antriebsmotors und einem Zustand einer Anzeigeleuchte besteht.

6. Elektrisches Bett system
nach Anspruch 5, wobei das Bettbrett mindestens eines umfasst, das aus der Gruppe ausgewählt ist, die aus einem Kopfbettbrett, einem Beinbettbrett, einem Hüftbettbrett und einem Hüftbettbrett besteht.

7. Elektrisches Bett system nach Anspruch 1, wobei der Server ein Cloud-Server ist.

8. Elektrisches Bettsystem
nach einem der Ansprüche 1-7, wobei die Endvorrichtung vom Server Informationsdaten empfängt und anzeigt, die den Zustand des elektrischen Bettes umfassen.

9. Elektrisches Bettsystem
nach einem der Ansprüche 1-7, wobei die Endvorrichtung von dem Server Informationsdaten empfängt und anzeigt, die den Zustand des elektrischen Bettes umfassen.

10. Elektrisches Bettsystem
nach einem der Ansprüche 1-7, wobei die Endvorrichtung mit dem Server über mindestens ein Verbindungsmittel kommuniziert, das aus der Gruppe bestehend aus Wi-Fi, 2G, 3G und 4G ausgewählt ist.

11. Elektrisches Bettsystem
nach Anspruch 8, wobei die Überwachungsdaten mindestens eines der folgenden Elemente umfassen:
Herzfrequenz, Atemfrequenz, Intensität des Schnarchens, Intensität der Atmung und Anzahl der Dreh- und Schwenkbewegungen im Bett.

12. Elektrisches Bettsystem
Endvorrichtung nach Anspruch 9, wobei der Zustand des elektrischen Bettes mindestens eines aus der Gruppe umfasst, die aus einem Neigungswinkel jeder Bettplanke, einem Zustand eines Antriebsmotors und einem Zustand einer Anzeigeleuchte besteht.

## Revendications

1. Un système de lit électrique intelligent, composé d'un lit électrique, d'un dispositif terminal, d'un serveur et un module de communication sans fil;
dans lequel le lit électrique est conçu pour surveiller en temps réel les conditions physiques d'un utilisateur et ajuster la position du lit électrique à partir des données de surveillance. Le lit électrique se compose de ce qui suit :
un module de surveillance (1), qui permet de surveiller les conditions physiques de l'utilisateur, et d'envoyer les données de surveillance au le module de communication sans fil (3) ; ainsi qu'un
module de commande principal (2), configuré pour recevoir un signal de commande du module de communication sans fil (3), et ajuster la position du lit électrique à partir du signal de commande ;
dans lequel le module de communication sans fil (3) transmet les données de surveillance recueillies au serveur, reçoit le signal de commande du serveur et envoie le signal de commande au module de commande principal (2) ; outre qu'il
est **caractérisé par le fait que**
le serveur génère le signal de commande à partir d'un signal de réglage envoyé par le dispositif terminal et des données de surveillance ; et
dans lequel le dispositif terminal est conçu pour régler l'état d'un lit électrique par l'intermédiaire du serveur, le dispositif terminal envoyant le signal de réglage au serveur ;
dans lequel le serveur génère, en fonction du signal de réglage, le signal de commande pour ajuster l'état du lit électrique, et envoie ensuite le signal de commande au lit électrique.

2. Le système de lit électrique
de la revendication 1, dans lequel le module de commande principal (2) envoie des données d'information qui comprennent l'état du lit électrique au module de communication sans fil (3), et le module de communication sans fil (3) envoie les données d'information au serveur.

3. Le système de lit électrique
de la revendication 1, dans lequel un moyen de connexion du module de communication sans fil (3) inclut au moins un élément sélectionné dans le groupe consistant en Wi-Fi, Bluetooth, infrarouge et ZigBee.

4. Le système de lit électrique
de la revendication 1, dans laquelle les données de surveillance incluent au moins une donnée sélectionnée dans le groupe comprenant la fréquence cardiaque, la fréquence respiratoire, l'intensité des ronflements, l'intensité de la respiration et le nombre de rotations et de basculements dans le lit.

5. Le système de lit électrique
de la revendication 1, dans laquelle la situation du lit électrique inclut au moins un élément sélectionné dans le groupe qui consiste en un angle d'inclinaison de chaque plan de lit, un état d'un moteur d'entraînement, et un état d'un voyant.

6. Le système de lit électrique
de la revendication 5, dans laquelle la planche de lit inclut au moins un élément sélectionné dans le groupe incluant une planche de lit pour la tête, une planche de lit pour les jambes, une planche de lit pour les fesses, et une planche de lit pour la taille.

7. Le système de lit électrique
de la revendication 1, dans laquelle le serveur s'apparente à un serveur dématérialisé (cloud)

8. Le système de lit électrique
de l'une quelconque des revendications 1 à 7, dans lesquelles le dispositif terminal reçoit du serveur et affiche des données de surveillance qui indiquent les conditions physiques d'un utilisateur du lit électrique.

9. Le système de lit électrique
dans l'une des revendications 1 à 7, dans laquelle le dispositif terminal reçoit du serveur et affiche des données d'information indiquant l'état du lit électrique.

10. Le système de lit électrique
de l'une quelconque des revendications 1 à 7, dans lesquelles le terminal communique avec le serveur au moyen d'au moins une connexion choisie dans le groupe constitué de Wi-Fi, 2G, 3G et 4G.

11. Le système de lit électrique
de la revendication 8, dans laquelle les données de surveillance incluent au moins une donnée sélectionnée dans le groupe comprenant la fréquence cardiaque, la fréquence respiratoire, l'intensité des ronflements, l'intensité de la respiration et le nombre de rotations et de basculements dans le lit.

12. Le système de lit électrique
de la revendication 9, dans laquelle la situation du lit électrique inclut au moins un élément sélectionné dans le groupe qui consiste en un angle d'inclinaison de chaque plan de lit, un état d'un moteur d'entraînement, et un état d'un voyant.
